# EUROPÄISCHE PATENTSCHRIFT

(11) **EP 2 192 984 B1**
(45) Veröffentlichungstag und Bekanntmachung des Hinweises auf die Patenterteilung: **12.06.2013**
(21) Anmeldenummer: 09733639.0
(22) Anmeldetag: 15.04.2009
(51) Int. Cl.: B01L 3/00, C12M 1/18, C12M 3/04

(54) **TEILAKTIVES MIKROFLUIDISCHES SYSTEM FÜR DIE 3D-ZELLKULTIVIERUNG SOWIE VERFAHREN ZU DESSEN PERFUSION**
PARTIALLY ACTIVE MICROFLUIDIC SYSTEM FOR 3D CELL CULTIVATION AND METHOD FOR PERFUSION THEREOF
SYSTÈME MICROFLUIDIQUE PARTIELLEMENT ACTIF POUR LA CULTURE CELLULAIRE EN 3D ET PROCÉDÉ POUR SA PERFUSION

(30) Priorität: 15.04.2008 DE 102008019691
(43) Veröffentlichungstag der Anmeldung: 09.06.2010
(73) Patentinhaber: Technische Universität Ilmenau, 98693 Ilmenau (DE)
(72) Erfinder: SCHOBER, Andreas, 90762 Fürth (DE); AUGSPURGER, Caroline, 98693 Ilmenau (DE); WEISE, Frank, 98693 Ilmenau (DE); FERNEKORN, Uta, 99094 Erfurt (DE); HILDMANN, Christian, 99085 Erfurt (DE); HAMPL, Jörg, 99087 Erfurt (DE)
(74) Vertreter: Engel, Christoph Klaus
(86) Internationale Anmeldenummer: PCT/EP2009/054444
(87) Internationale Veröffentlichungsnummer: WO 2009/127647

(56) Entgegenhaltungen:
- WO-A1-93/07258
- DE-U1- 9 215 941
- US-A1- 2004 171 169
- US-A1- 2005 191 759

## Beschreibung

Die Erfindung betrifft kostengünstig herzustellende, parallele mikrofluidische Systeme zur Kultivierung von fortgeschrittener Zellkultur (3D-Kultur, Stammzellen etc.), die in jedem biotechnologisch und biomedizinisch arbeitenden Labor sinnvoll eingesetzt werden können und zudem HTS-fähig (HTS = High-Throughput-Screening) sind. Solche Systeme sind prinzipiell geeignet, in der Wirkstoffsuche bei ADME/Tox Screenings eingesetzt zu werden und weisen daher ein hohes Vermarktungspotential auf. ADME/Tox Studien dienen der Untersuchung von Substanzen auf ihre Eigenschaften im (menschlichen) Organismus in Bezug auf Absorption, Distrubution, Metabolisierung, Exkretion und Toxizität.

Für vielfältige Anwendungen in der Medizin und Pharmaforschung oder in der Kosmetikindustrie ist die Kultivierung biologischen Materials eine herausragende Aufgabe. Hierbei ist Zellmaterial, das können Gewebeschnitte z. B. aus Biopsien oder primäre Zellen, die aus Tieren oder Menschen gewonnen wurden, Zelllinien oder gentechnisch veränderte Zellen sein, möglichst so zu kultivieren, dass seine natürliche Funktion und Lebensfähigkeit beibehalten werden bzw. die angestrebte Funktion möglichst optimal umgesetzt wird.

Die Bandbreite der Anwendung reicht dabei vom Bereitstellen von Testsystemen für die Pharma- und Kosmetikforschung über die medizinische Forschung (z.B. die Stammzellforschung) bis zur Produktion von Impfstoffen und der Grundlagenforschung. Ein wichtiger Aspekt ist dabei sowohl der sparsame Umgang mit wertvollem Zellmaterial als auch die Bereitstellung vieler verschiedener paralleler Ansätze zum Durchtesten von unterschiedlichen Parametern.

In einem Gewebe müssen Nährstoffe und Substanzen zur Differenzierung (z. B. Botenstoffe) in ausreichendem Maße zur Verfügung gestellt werden. Dies kann bei aus mehreren Zellschichten bestehenden Geweben (3D-Zellkultur) nicht mittels reiner Diffusion gewährleistet werden, sondern bedarf auch aktiver Transportmechanismen analog zum Blutkreislauf (und auch dem Lymphsystem), durch welche die benötigten Substanzen interzellulär zu- und abgeführt werden. Es hat sich gezeigt, dass hierbei die Geometrie z. B. von Unterstützungssystemen, Gerüsten zur Ansiedelung von Zellen, der Abstand und die Führung von fluidischen Versorgungskanälen sowie die Dichte, die Art und der Typus der Zellbesiedelung wichtige Parameter sind, die über die erfolgreiche Kultivierung bzw. Herstellung von Geweben im Tissue Engineering entscheiden. Zudem ist die Induktion von Differenzierungsprozessen durch gezielte Wirkstoffabgabe in einem Gewebe ein ungelöstes Problem, das bislang nur über "trial and error" Methoden getestet wird.

Für die ordnungsgemäße Funktion eines Gewebes oder Organs sind viele Parameter von Bedeutung. Um die Aufrechterhaltung von Organ- und Gewebefunktionen *in vitro* zu gewährleisten, muss nicht nur die korrekte molekulare, sondern vor allem auch die korrekte makroskopische Architektur des Zellverbandes reproduziert werden. So ist für viele Anwendungen essentiell, dass verschiedene Zellarten gemeinsam kultiviert werden (Kokultur), weil etwa eine Zellart Stoffe produziert, die die andere Zellart beeinflussen, z. B. zur Differenzierung anregen. Bei anderen Anwendungen ist man etwa daran interessiert, die Interaktionen der Zellarten oder die gerichtete Verstoffwechslung durch die Zellarten in Reihe zu untersuchen. Weiterhin ist bekannt, dass primäre Zellen häufig ihre zelltypspezifischen (differenzierten) Funktionen verlieren, wenn sie als Monolayer (einschichtig) kultiviert werden. Es sind deshalb verschiedene Anstrengungen unternommen worden, Zellkultursysteme zu entwickeln, die die dreidimensionale in vivo-Situation des korrespondierenden Gewebes besser nachbilden.

Eine Möglichkeit besteht in der mikrofluidisch unterstützten Kultivierung von Zellen in 3D-Kultur. Dabei wird von 3D-Kulturen in mikrostrukturierten Trägern (3D-Zellkultur-Probenträger - auch 3D-CellChips genannt) ausgegangen, die sich bereits in der Erprobung auch für eine Langzeit-Kultivierung befinden. Als 3D-Zellkultur-Probenträger (z.B. Matrigrid) wird dabei eine flächige oder drei dimensional ausgeführte Unterstützungsstruktur zur dreidimensionalen Kultivierung von Zellen, die vorzugsweise perforiert ist und somit mit Medium durchströmt werden kann, bezeichnet.

Ein solcher Probenträger (3D-CellChip) ist z.B. aus der WO 93/07258 bekannt, wobei sich die Größenordnungen für die Stützgerüste in der fortgeschrittenen Zellkultur an den physiologischen Größen orientieren. Die Höhe des Stützgerüstes für Zellen in einer dreidimensionalen Kultur, die von beiden Seiten versorgt wird, sollte 300 µm nicht übersteigen, wenn die Zellschicht nicht aktiv durchströmt wird. Der Prototyp des beschriebenen Zellträgers beherbergt auf einer Fläche von 1 cm² ca. 900 (30 x 30) Microcontainer mit den Dimensionen 300 µm x 300 µm x 300 µm (L x B x H) bei einer Wandstärke von 50 µm. Der Boden besitzt Poren, die einen ungehinderten Stoffaustausch schon bei Superfusion (Über- bzw. Unterströmen) gewährleisten, aber auch das Durchströmen des Zellverbandes mit Medium erlauben (Perfusion).

Der Einsatz von Mikrosystemtechniken zur Herstellung von Bioreaktoren hat sich als großer Vorteil zur definierten Kultivierung von fortschrittlichen Zellkulturen herausgestellt. Die experimentell notwendige Diversität bezüglich verschiedener Zelllinien, Seren, Medien und Wirkstoffe kann ökonomisch nur durch die Anwendung miniaturisierter Systeme adressiert werden.

Aus der Dissertation von C. Augspurger (Leipzig 2007) ist bekannt, dass derartige Probenträger standardmäßig in Bioreaktoren mit einigen 25 ml Volumen mit externen Pumpen als in sich geschlossene Systeme betrieben werden. Diese Bioreaktoren sind jedoch nur eingeschränkt für Anwendungen u.a. in der Pharmaforschung einsetzbar, da sie nicht kompatibel zu den Schnittstellen der automatisierten Labortechnik wie z.B. dem Mikrotiterplattenformat (oder auch "MTP footprint") sind. Außerdem ist es nicht möglich, zu diesen Bioreaktoren Testsubstanzen automatisch zuzuführen. Sie sind zudem durch eine mangelnde Parallelisierbarkeit charakterisiert. Ein weiteres Problem besteht darin, dass das eingesetzte notwendige biologische Material bzw. Volumen für viele Aufgaben einfach zu groß ist, um sinnvoll für parallele Ansätze eingesetzt zu werden.

Für die angestrebte Miniaturisierung und Parallelisierung auf Mikrotiterplattenbasis ist das Format und Volumen gerade für Screening Anwendungen zu groß und aufwendig. Daher wird in verschiedenen Ansätzen das Einbringen von 3D strukturierten Polymeren oder Polymerschäumen in 24- oder 96-er Mikrotiterplatten als Supportstrukturen durchgeführt (s.a. Wintermantel "Dreidimensionale Zellkulturen"; TUM- Mitteilungen 4-2006 (Oktober 2006). Diese Systeme verfügen jedoch über keine aktive Perfusion. Ein aktives Durchströmen des Gewebe- und Zellmaterials ist also nicht möglich. Deshalb stellen diese Systeme ein unzureichendes Modell für die Nachbildung der Organe im lebenden Organismus dar.

Aus der US 6,943,009 B2 ist eine Vorrichtung für Zellkultivierung bekannt, die an ihrer Oberseite zusätzliche Öffnung zur Zugabe- und Abnahme von Lösungen aufweist. Die Vorrichtung besitzt jedoch keine integrierte 3D-Strukturierung für eine durchströmte 3D-Zellkultur und ist somit nicht für die *in vivo* nahe Zellkultivierung geeignet. Zudem kann bei diesem System nur jeweils ein Netz pro well eingehängt werden (die Netze sind nicht stapelbar), was Kokulturen von verschiedenen in Reihe geschalteten Zelltypen im gleichen well, aber in unterschiedlichen Kompartimenten nicht erlaubt.

Die US 2005/0191759 A1 zeigt ein Gerät und ein Verfahren zur Ausführung einer Flüssigphasen-Mikroextraktion. Das Gerät umfasst eine Flüssigmembran und einen optionalen Träger auf einem porösen Polymersubstrat, welches eine Hohlfaser sein kann.

In der DE 602 16 076 T2 ist eine Hohlfasermembran-Mehrbehälterplatte zum Anreichern und Reinigen von Proben beschrieben, die eine Vielzahl von Behältern zum Aufnehmen mehrere Proben besitzt. Weiterhin sind mehrere Hohlfasern vorgesehen, von denen sich jeweils eine in diesen Behältern befindet und jeweils eine flüssige Extraktionsmembran besitzt, die einen inneren Hohlraum der Hohlfaser umschließt. Eine Kultivierung von Zellen und Geweben in einer *in vivo* Situation lässt sich mit dieser Platte jedoch nicht erreichen.

Generell zeigen bekannte Inlay-Systeme zum Teil ähnliche Probleme wie der o.g. Ansatz von Wintermantel et al., d.h. eine aktive Perfusion ist in einer solchen Mikrotiterplatte nicht möglich, es sei denn, mit einem Pipettiersystem wird Flüssigkeit hinzu- oder abgegeben. Hier zeigen sich aber fundamentale Probleme. Durch den Flüssigkeitsstrom können die Zellen abgelöst werden, d.h. der Zellverband kann aufschwimmen und vor allem kann auf diese Weise keine zirkuläre Durchströmung durchgeführt werden. Ein weiteres Problem von Inlay-Systemen ist, dass beim Einbringen von mit Zellen besiedelten, vorkultivierten Inlays, die Gefahr des Ablösens des Zellverbands ebenso durch den Strömungsdruck beim Eintauchen relevant wird.

Aufgabe der vorliegenden Erfindung ist es deshalb, die aus dem Stand der Technik bekannten Nachteile zu überwinden und auf der Basis konventioneller Mikrotiterplatten ein teilaktives mikrofluidisches System für die 2D- und/oder 3D-Zellkultivierung für die Laborautomatisierung bzw. den automatisierten High-Content-Screening (HCS), also der automatischen Bestimmung vieler biologischer und physikalischer Parameter, bzw. den sogenannten High-Throughput-Screening (HTS) bereitzustellen, ohne die Gefahr des Aufschwimmens in eine mit Flüssigkeit befüllte Mikrotiterplatte einzubringen. Ein solches System soll auch stapelbar sein (z.B. für eine Kokultur).

Diese Aufgabe wird durch ein teilaktives mikrofluidisches System gemäß dem beigefügten Anspruch 1 und durch ein Verfahren gemäß dem beigefügten nebengeordneten Anspruch 9 gelöst.

Eine Möglichkeit, ein teilaktives mikrofluidisches System zur 3D-Zellkultivierung zu realisieren, besteht in der Integration von Membraninlays, vorzugsweise als eine flächige oder dreidimensionale Unterstützungsstruktur zur dreidimensionalen Kultivierung von Zellen ausgeführt, in eine Mikrotiterplatte.

Eine vorteilhafte Ausgestaltung des erfindungsgemäßen Systems wird durch die Integration eines Saugsystems und eines Sammelkompartiments erreicht.

Die Erfindung wird im Folgenden anhand der Zeichnung näher erläutert. Es zeigen:
- Fig. 1: eine Ausführungsform eines teilaktiven mikrofluidischen Systems für eine 3D-Zellkultivierung (Stand der Technik)
- Fig. 2: eine erfindungsgemäße Ausführungsform des teilaktiven mikrofluidischen Systems für die 3D-Zellkultivierung;
- Fig. 3: eine erfindungsgemäße Ausführungsform des teilaktiven mikrofluidischen Systems für die 3D-Zellkultivierung; und
- Fig. 4: eine prinzipielle Darstellung eines erfindungsgemäßen Verfahrens zur Perfusion eines teilaktiven mikrofluidischen Systems;
- Fig. 5-9: Detaildarstellungen verschiedener Ausführungsformen von Formkörpern, die in einzelne Well einer Mikrotiterplatte einsetzbar sind;
- Fig. 10: eine besondere Ausführungsform des erfindungsgemäßen Systems, bei welcher Saugsystem und eine Sammelkompartiment integriert sind.

Die vorliegende Erfindung nutzt zur Lösung der o.g. Aufgabe jeweils eine Öffnung in einem Inlay, vorzugsweise eine Ausgleichkapillare, die nicht in direktem Kontakt mit der aufnehmenden Kavität in einem Well des teilaktiven mikrofluidischen Systems (Mikrotiterplatte) steht. Das Inlay formt eine Kavität innerhalb des Well zur Aufnahme einer Zellkultur. Durch die Kapillaröffnungen wird ein Druckausgleich realisiert. Zusätzlich dienen sie als Einfüllführung für Kapillarspitzen von Pipettiersystemen, vorzugsweise von Pipettierrobotern, die durch periodische Aufnahme und Abgabe von Reagenz eine aktive Durchströmung eines solchen Systems simulieren können.

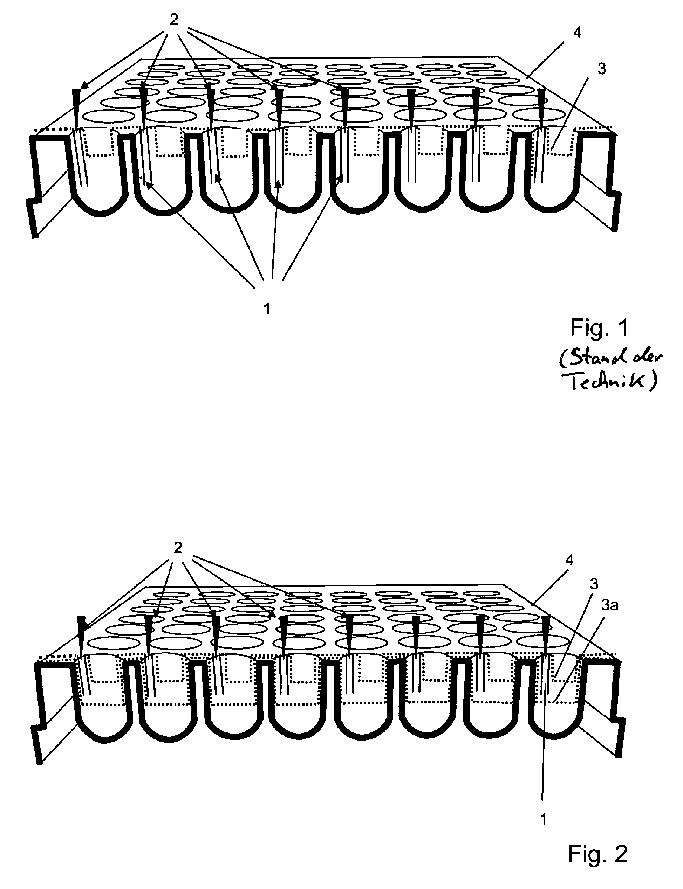

Fig. 1 zeigt in vereinfachter Darstellung eine Ausführungsform aus dem Stand der Technik eines teilaktiven mikrofluidischen Systems für eine 3D-Zellkultivierung. Durch Kapillaröffnungen 1 kann z.B. mit Hilfe von Pipettierspitzen 2 Flüssigkeit eingebracht werden. Die Kapillaröffnungen 1 ermöglichen zudem schon mit Zellen besiedelte Inlays 3, die einen porösen 3D-Zellkulturträger bereitstellen.

In Fig. 2 ist eine Ausführungsform dargestellt, die eine Lösung für Kokultivierungsaufgaben bereit stellt. Erfindungsgemäß können dabei auch mehrere Inlays 3, 3a in die Mikrotiterplatte 4 eingebracht werden. Hierbei verfügt nur das oberste Inlay 3 über die Öffnungen 1. Durch diese spezielle Ausführungsform kann für anhaftende Zellen ein Anströmen realisiert oder für frei schwimmende Zellen ein Reaktionsvolumen geschaffen werden.

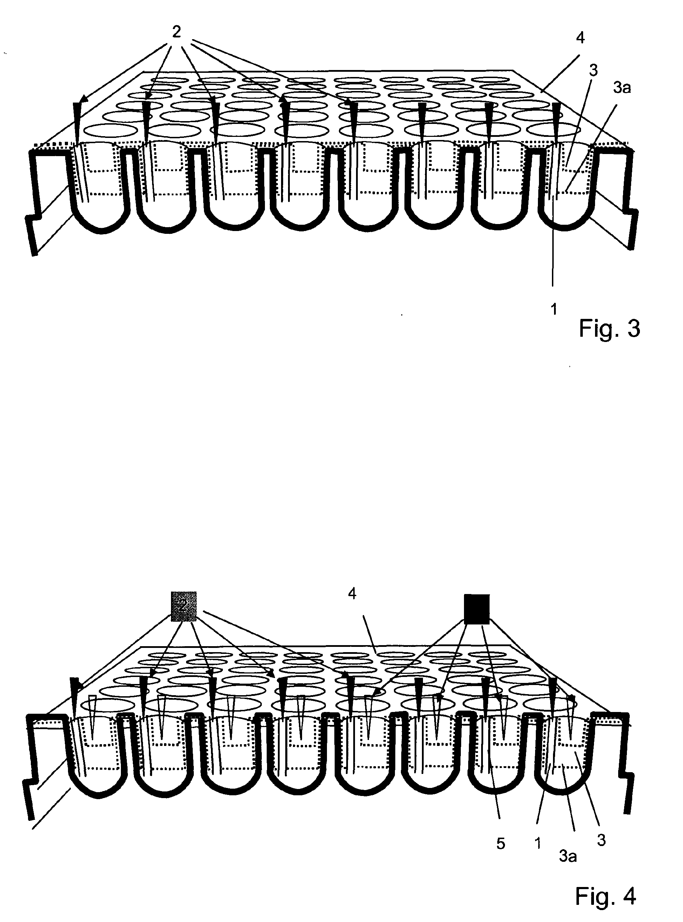

Gemäß der in Fig. 3 gezeigten abgewandelten Ausführungsform ist es ebenso möglich, dass alle in die Mikrotiterplatte 4 integrierten Inlays 3, 3a die Öffnungen 1 aufweisen, die jeweils miteinander verbunden sind. Dadurch kann das Aufschwimmen der Zellen im unteren Bereich des Mikrotiterplattenwells verhindert werden.

In Fig. 4 ist das erfindungsgemäße Verfahren prinzipiell dargestellt. Ein zyklisches Zupipettieren über die Pipettierspitzen 2, z.B. in eine auf den Well-Boden führende Kapillare 5, und ein Abnehmen über weitere Pipettierspitzen 6, die z.B. nur oberhalb des obersten Inlays 3 eingetaucht werden, erzeugt einen pulsierenden Flüssigkeitsstrom und simuliert ein Pumpensystem. Auf diese Weise wird erreicht, dass Flüssigkeit durch den porösen 3D-Zellkulturträger nachströmt und die Versorgung der Zellen gewährleistet wird. Der Pipettierzyklus kann auch in umgekehrter Richtung erfolgen.

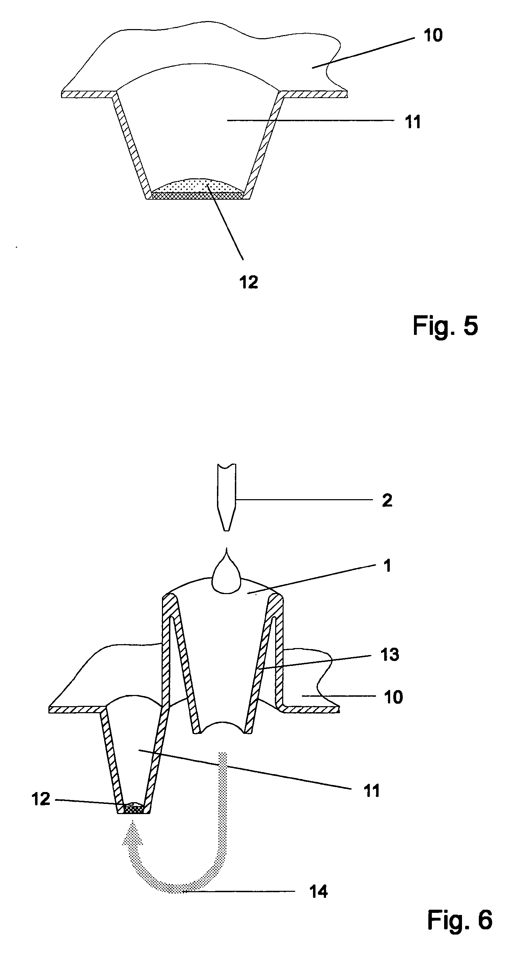

Die Figuren 5 bis 9 zeigen Detaildarstellungen verschiedener Ausführungsformen von Formkörpern 10, welche die Inlays 3 ausbilden. Die geformten Kavitäten werden in die einzelnen Wells der hier nicht dargestellten Mikrotiterplatte eingesetzt. Der Formkörper 10 kann unterschiedlich tief geformt sein und unterschiedliche Durchmesser haben. Der Formkörper muss ausreichend Platz für eine 3D-Zellkultivierung und ausreichend Medium im gleichen Kompartiment bereitstellen. Wenn der Formkörper in ein Well einer 96-well-Mikrotiterplatte eingehängt werden soll, hat er vorzugsweise eine Durchmesser, der leicht unter dem des Wells liegt. Werden mehrere Formkörper 10 gestapelt (siehe unten), so können sie zur besseren Stapelbarkeit unterschiedlich geformt sein (etwa mit abnehmendem Durchmesser).

Gemäß Fig. 5 bildet der Formkörper 10 zunächst die Kavität bzw. ein Probenkompartiment 11. Die Unterseite des Probenkompartiments 11 ist durch einen porösen Probenträger 12 geschlossen, auf welchem die Zellkultur aufgebracht ist. Die Seitenwände des Formkörpers 10 sind je nach Anwendung porös (es erfolgt ein ungerichteter Stofftransport) oder nicht porös (für gerichteten Stofftransport) ausgebildet.

Der Probenträger 12 und/oder die Seitenwände des Formkörpers besitzen eine zweidimensional oder dreidimensional strukturierte Siebstruktur, vorzugsweise begrenzt auf die Fläche im Bodenbereich. Es ist entscheidend, dass der einzuhängende Formkörper 10 an seiner tiefsten Stelle (hier Probenträger 12) nicht nur eine Siebstruktur darstellt (mit Porendurchmessser kleiner als Zelldurchmesser, vorzugsweise kleiner 5µm), sondern auch in einer für die Zellkultur geeigneten Weise strukturiert ist, vorzugsweise dreidimensional (etwa mit Vertiefungen, als Schaum, oä), da der Formkörper der Kultivierung von Zellen dient und eine 3D-Strukturierung in vielen Fällen Vorteile bei der Erhaltung oder Generierung der zellulären Differenzierung bietet. Eine 2D-Strukturierung könnte eine physikalische oder chemische Modifizierung/Musterbildung sein, in der Weise, dass Zellen besser, schlechter oder regional unterschiedlich adhärieren/anheften, je nach Anwendung.

Die erwähnte Siebstruktur bedeutet dabei, dass die Poren zwar kleiner als der Zelldurchmesser sein müssen, dass sie aber auch groß genug sein müssen, um einen Mediumfluss von Kompartiment zu Kompartiment zu erlauben. Entnimmt man beispielsweise die Flüssigkeit aus einem Formkörper 10, der derart in Flüssigkeit getaucht und dort fixiert ist, dass nun der Flüssigkeitspegel höher steht als der Boden des eingehängten Formkörpers, so soll die Flüssigkeit aufgrund des hydrostatischen Druckes und zum Teil auch aufgrund von Kapillarkräften durch die Siebstruktur im Boden des Formkörpers 10 in das vom Formkörper begrenzte Kompartiment 11 einströmen. Gleiches erreicht man, wenn man nicht im Formkörper die Flüssigkeit zuerst entnimmt, sondern in dem umgebenden Flüssigkeitsreservoir durch Flüssigkeitszugabe einen hydrostatischen Druck aufbaut. Dazu kann das Volumen des umgebenden Flüssigkeitsreservoirs auch größer als das Volumen des Probenkompartiments ausgebildet sein. Die Siebstruktur dient aber nicht nur der Durchströmung, sondern auch als Zellkultursubstrat/Probenträger, vorzugsweise als 3D-Zellkultursubstrat. Sie stellt somit nicht nur eine einfache Membran dar, sondern mindestens eine 3D-strukturierte Membran oder einen porösen Schaum oä.

Wie sich aus Fig. 6 erkennen lässt, ist am Formkörper 10 vorzugsweise ein Pipettierabschnitt 13 angeformt, welcher die Öffnung 1 bereit stellt, in welche ein Medium über die Pipettierspitze 2 eingebracht werden kann. Wenn der Pipettierabschnitt oberhalb des Probenträgers 12 mündet, ergibt sich bei der Zugabe von Medium ein durch den Strömungspfeil 14 symbolisierte Strömungsrichtung des Mediums, die einer Perfusion der Zellkultur bewirkt.

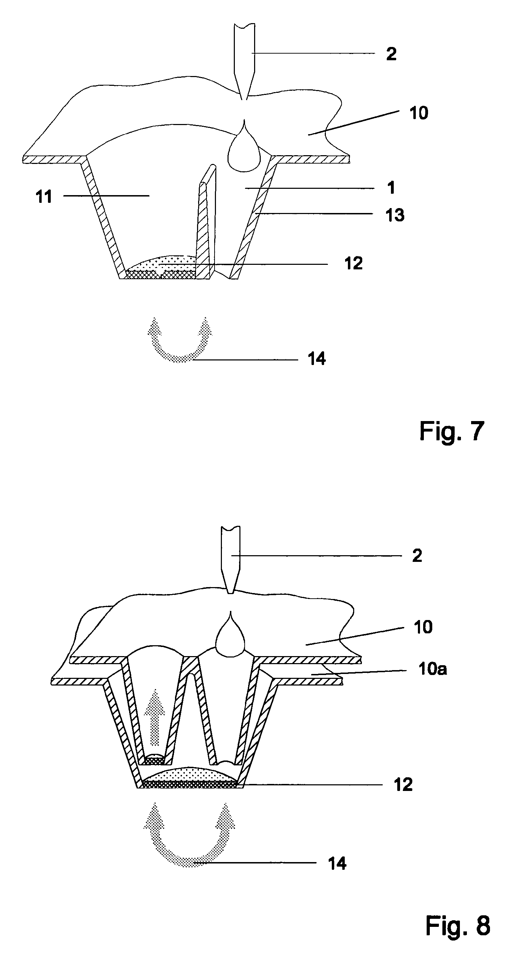

Fig. 7 zeigt eine abgewandelte Ausführungsform, bei welcher der Pipettierabschnitt etwa in der Ebene des Probenträgers 12 mündet. Je nach Zufuhr bzw. Entnahme von Medium kann über den eingestellten Flüssigkeitspegel im Pipettierabschnitt die Strömungsrichtung des Mediums wechseln.

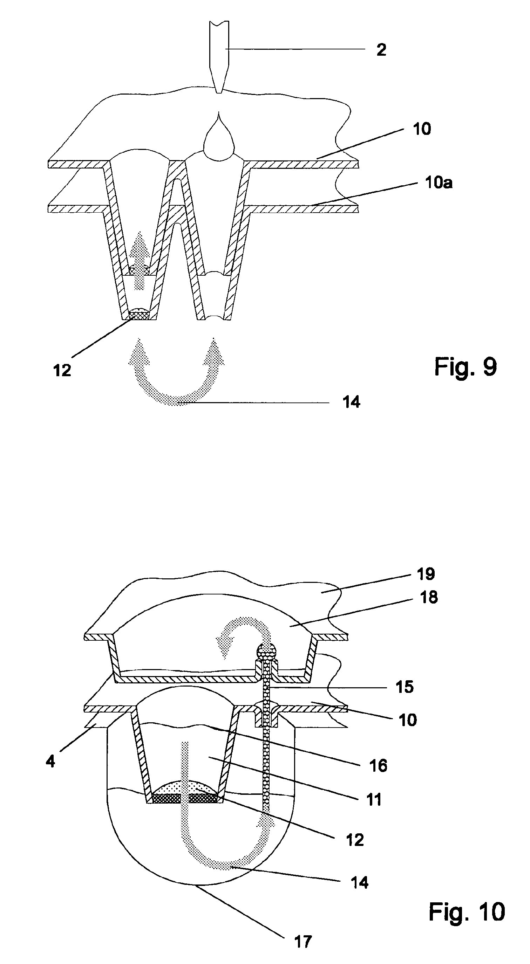

Die Figuren 8 und 9 zeigen Varianten zur Stapelung mehrere Formkörper 10, 10a. Dies ermöglicht mehrere Ebenen der Zellkultur, womit das Anwendungsspektrum des Erfindungsgegenstandes erhöht wird (z.B. verschiedene Zellarten je Kulturebene). In Fig. 10 ist eine besondere Ausführungsform des Systems dargestellt, bei welcher eine Integration eines Saugsystems und eines Sammelkompartiments vorgenommen ist. Das Saugsystem besteht dabei aus einem fasrigen oder porösen Material, das durch sehr hohe Kapillarkräfte gekennzeichnet ist und einen Docht 15 bildet. Wird nun in das groß dimensionierte Probenkompartiment 11 eine hoher Pegelstand 16 an Medium gegeben und der Docht 15 bis in das in einem umgebenden Well 17 (oder einem anderen Flüssigkeitsbehälter) befindliche Medium getaucht, wird durch die hohen Kapillarkräfte im Saugsystem 15 das Medium in ein Sammelkompartiment 18 eines zweiten Formkörpers 19 gefördert. Dieser Effekt kann durch die Füllung des Sammelkompartiment 18 mit einem stark aufsaugendem Material verstärkt werden. Durch Dimensionierung des Saugsystems 15 lässt sich die gewünschte Förderleistung einstellen. Es kann so über einen längeren Zeitraum eine Durchströmung des Probenträgers 12 ohne jegliche Hilfsmittel oder aktive Komponenten realisiert werden. Diese aktive Durchströmung führt unweigerlich zu einer besseren Versorgung der Zellen. Somit bietet diese Ausführungsform einen weiteren signifikanten Vorteil gegenüber bekannten Systemen.

Ein erfindungsgemäßes teilaktives mikrofluidisches System ist in allen Volumen und Größenbereichen, die durch Pipettierroboter erreicht werden können, realisierbar.

### Bezugszeichenliste

| | |
|---|---|
| 1 | Öffnungen, vorzugsweise Kapillaröffnungen |
| 2 | Pipettierspitzen zum Zupipettieren |
| 3, 3a | Inlay |
| 4 | Mikrotiterplatte |
| 5 | Kapillare |
| 6 | Pipettierspitzen zum Absaugen |
| | |
| 10, 10a | Formkörper |
| 11 | Probenkompartiment |
| 12 | Probenträger |
| 13 | Pipettierabschnitt |
| 14 | Strömungspfeil |
| 15 | Docht / Saugsystem |
| 16 | Pegelstand im Probenkompartiment |
| 17 | Well |
| 18 | Sammelkompartiment |
| 19 | zweiter Formkörper |

## Patentansprüche

1. Teilaktives mikrofluidisches System für die Zellkultivierung umfassend ein Multiwellsystem (4) mit mehreren Wells (17), in denen Zellen aufnehmende Kavitäten gebildet sind, wobei jede Kavität (11) durch mindestens ein Inlay (3) gebildet ist, welches eine Öffnung (1) aufweist, die nicht direkt in die Kavität (11), sondern in einen die Kavität umgebenden Raum (17) mündet, wobei das im umgebenden Raum vorhandene Medium über eine Siebstruktur im Inlay (3) mit dem in der Kavität (11) enthaltenen Medium kommuniziert, **dadurch gekennzeichnet, dass** das System mehrere, übereinander gestapelte Inlays (3) aufweist, wobei zumindest das oberste Inlay mit den Öffnungen (1) versehen ist.

2. Teilaktives mikrofluidisches System nach Anspruch 1,
**dadurch gekennzeichnet, dass** die Inlays (3) als flächige oder dreidimensionale Unterstützungsstrukturen zur dreidimensionalen Kultivierung von Zellen ausgeführt sind.

3. Teilaktives mikrofluidisches System nach Anspruch 1 oder 2,
**dadurch gekennzeichnet, dass** die Öffnungen durch an den Inlays (3) angeformte Kapillaröffnungen (1) gebildet sind.

4. Teilaktives mikrofluidisches System nach einem der Ansprüche 1 bis 3, **dadurch gekennzeichnet, dass** die Öffnungen (1) in Pipettierabschnitten (13) angeordnet sind, die getrennt von den Kavitäten (11) geformt sind.

5. Teilaktives mikrofluidisches System nach einem der Ansprüche 1 bis 4, **dadurch gekennzeichnet, dass** die Inlays (3) durch Formkörper (10) gebildet sind, die mindestens ein Probenkompartiment (11) als Kavität formen, wobei mindestens der Boden des Probenkompartiments (11) als Probenträger (12) mit Siebstruktur gebildet ist.

6. Teilaktives mikrofluidisches System nach Anspruch 5,
**dadurch gekennzeichnet, dass** die Formkörper (10) zumindest abschnittsweise eine zweidimensional oder dreidimensional strukturierte Siebstruktur aufweisen.

7. Teilaktives mikrofluidisches System nach Anspruch 5 oder 6,
**dadurch gekennzeichnet, dass** die Formkörper in eine Mikrotiterplatte (MTP) eingehängt und mit einem Deckel verschlossen sind.

8. Teilaktives mikrofluidisches System nach einem der Ansprüche 1 bis 7, **dadurch gekennzeichnet, dass** alle Inlays (3) mit den Öffnungen (1) versehen sind, wobei die Öffnungen (1) der einzelnen Inlays miteinander verbunden sind.

9. Verfahren zur Perfusion eines teilaktiven mikrofluidischen Systems nach einem der Ansprüche 1 bis 8, **dadurch gekennzeichnet, dass** Flüssigkeit mit Hilfe eines Pipettierwerkzeugs in die Öffnungen (1) der Inlays (3) hineinpipettiert und oberhalb der Inlays (3) entnommen wird.

## Claims

1. Partially active microfluidic system for cell cultivation comprising a multiwell system (4) with several wells (17), in which cell receiving cavities are formed, whereat each cavity (11) is formed by at least one inlay (3) which has an opening (1) that opens not directly into the cavity (11) but into a space (17) surrounding the cavity, where the medium present in the surrounding space communicates via a sieve structure in the inlay (3) with the medium contained in the cavity (11), **characterized in that** the system has several inlays (3) stacked on top of each other, where at least the uppermost inlay is provided with the openings (1).

2. Partially active microfluidic system according to Claim 1, **characterized in that** the inlays (3) are designed as two-dimensional or three-dimensional support structures for the three-dimensional cultivation of cells.

3. Partially active microfluidic system according to Claim 1 or 2, **characterized in that** the openings are formed by capillary openings (11) which are formed on the inlays (3).

4. Partially active microfluidic system according to one of Claims 1 to 3, **characterized in that** the openings (1) are arranged in pipetting sections (13) which are formed separately from the cavities (11).

5. Partially active microfluidic system according to one of Claims 1 to 4, **characterized in that** the inlays (3) are formed by molds (10) which form at least one sample compartment (11) as a cavity, where at least the bottom of the sample compartment (11) is formed as a sample support (12) with sieve structure.

6. Partially active microfluidic system according to Claim 5, **characterized in that** the molds (10) present at least in sections a two-dimensionally or three-dimensionally structured sieve structure.

7. Partially active microfluidic system according to Claim 5 or 6, **characterized in that** the molds are suspended in a microtiter plate (MTP), and closed with a cover.

8. Partially active microfluidic system according to one of Claims 1 to 7, **characterized in that** all the inlays (3) are provided with the openings (1), where the openings (1) of the individual inlays are connected to each other.

9. Method for the perfusion of a partially active microfluidic system according to one of Claims 1 to 8, **characterized in that** fluid is pipetted by means of a pipetting tool into the openings (1) of the inlays (3), and removed above the inlays (3).

## Revendications

1. Système microfluidique partiellement actif pour la culture cellulaire, comprenant un système multipuits (4) comportant plusieurs puits (17) dans lesquels sont formées des cavités recevant des cellules, chaque cavité (11) étant formée par au moins un insert (3) qui présente un orifice (1) qui ne débouche pas directement dans la cavité (11) mais dans un espace (17) entourant la cavité, le fluide se trouvant dans l'espace environnant communiquant par une structure de tamis située dans l'insert (3) avec le fluide contenu dans la cavité (11), **caractérisé en ce que** le système présente plusieurs inserts empilés (3), au moins l'insert supérieur étant pourvu des orifices (1).

2. Système microfluidique partiellement actif selon la revendication 1, **caractérisé en ce que** les inserts (3) se présentent sous forme de structures de soutien planes ou tridimensionnelles pour la culture tridimensionnelle de cellules.

3. Système microfluidique partiellement actif selon la revendication 1 ou 2, **caractérisé en ce que** les orifices sont constitués par des orifices capillaires (1) formés au niveau des inserts (3).

4. Système microfluidique partiellement actif selon une des revendications 1 à 3, **caractérisé en ce que** les orifices (1) sont disposés dans des sections de prélèvement (13) qui sont formées séparément des cavités (11).

5. Système microfluidique partiellement actif selon une des revendications 1 à 4, **caractérisé en ce que** les inserts (3) sont constitués par des corps moulés (10) qui constituent sous forme de cavités au moins un compartiment à échantillon (11), au moins le fond du compartiment à échantillon (11) étant constitué sous forme d'un porte-échantillon (12) à structure de tamis.

6. Système microfluidique partiellement actif selon la revendication 5, **caractérisé en ce que** les corps moulés (10) présentent au moins par sections une structure de tamis bidimensionnelle ou tridimensionnelle.

7. Système microfluidique partiellement actif selon la revendication 5 ou 6, **caractérisé en ce que** les corps moulés sont accrochés dans une plaque de microtitrage (MTP) et fermés par un couvercle.

8. Système microfluidique partiellement actif selon une des revendications 1 à 7, **caractérisé en ce que** tous les inserts (3) sont pourvus des orifices (1), les orifices (1) des différents inserts étant reliés entre eux.

9. Procédé de perfusion d'un système microfluidique partiellement actif selon une des revendications 1 à 8, **caractérisé en ce que** du liquide est incorporé à l'aide d'un outil à pipette dans les orifices (1) des inserts (3) ou prélevé au-dessus des inserts (3).
